# EUROPEAN PATENT APPLICATION

(11) **EP 1 586 557 A1**
(43) Date of publication of application: **19.10.2005**
(21) Application number: 03785474.2
(22) Date of filing: 30.12.2003
(51) Int. Cl.: C07C 311/08, A61K 31/18, A61K 47/40, A61K 47/48, A61P 29/00

(54) **4-NITRO-2- (4 -METHOXY)-PHENOXY -METHANESULFONANILIDE DERIVATES AND THEIR PHARMACEUTICAL USE**

(30) Priority: 31.12.2002 CN 02159419
(71) Applicant: Institute of Radiation Medicine, Academy of Military Medical Sciences Pla, Beijing 100850 (CN); Beijing Lu Yin Li Hua Pharmaceutical Science Technology Deevlopment Company, Ltd., Beijing 102600 (CH)
(72) Inventor: SUN, Zhuangrong, Beijing 100850 (CN); WU, Zuze, Beijing 100850 (CN); TANG, Zhongxiong, Beijing 100850 (CN); LIU, Aiquan, Beijing 100850 (CN)
(74) Representative: Minderop, Ralph H.
(86) International application number: PCT/CN2003/001145
(87) International publication number: WO 2004/058697

(57) **Abstract**

The present invention discloses 4-nitro-2-[(4'methoxy)-phenoxy] -methanesulfonyl aniline, or pharmaceutically acceptable salts, solvates or hydrates thereof, or mixtures thereof with povidone, phospholipid or cyclodextrin, and their pharmaceutical uses, especially as non-steroid anti-inflammatory and analgesic drugs.

## Description

### Technical Field

The present invention relates to derivatives of 4-nitro-2-(4'methoxy-phenoxy)-methanesulfonyl aniline and their pharmaceutical uses, especially in the preparation of anti-inflammatory and analgesic drugs.

### Background Art

Non-steroid anti-inflammatory drugs (NSAIDs) possess anti-inflammatory and analgesic effects, and have been extensively used clinically in treatment of various inflammations (such as osteoarthritis, inflammation of respiratory tract), tumor, thromboangiitis, postoperative pain, dysmenorrhea, diseases of the ear, nose and throat, and post-traumatic pain, inflammation, fever and other symptoms, in particular, they are more commonly used in arthritis. The role of prostaglandin in the induction of inflammation and the enzyme for its synthesis, cyclooxygenase, were discovered in the 1970s. Of course, the beneficial actions of prostaglandin have also been confirmed, such as providing protection for cells of the gastrointestinal tract, maintaining normal renal function, facilitating platelet aggregation, and the like. It was discovered later that cyclooxygenases can be classified into 2 groups, i.e. COX₁ and COX₂. COX₂ is mostly located in inflammation sites. Therefore, COX₂ inhibitors can affect the generation of prostaglandin in said location, resulting in anti-inflammatory and analgesic effects, with greatly reduced side-effects such as gastrointestinal ulcer and bleeding. In addition, they can inhibit and clear harmful superoxide radicals, hydrogen peroxide radicals, inhibit the synthesis of platelet activating factor, the release of tumor necrosis factor- α, the proteolytic enzymes, and the release of histamine, thus contributing to the anti-inflammatory and analgesic effects. Their anti-inflammatory, analgesic and antipyretic effects have been demonstrated in various experimental models. Therefore, NSAIDs, which belong to COX₂ inhibitors, are commonly used in clinical context. Among them, nimesulide, a selective COX₂ inhibitor, is extensively used for its remarkable therapeutic effects and low toxic side-effects. However, in recent years, there have been a number of reports worldwide of severe liver damage associated with administration of nimesulide. Therefore, there is still a need for developing new COX₂ inhibitors with even better therapeutic effects and lower side-effects.

### Disclosure of the invention

The purpose of the present invention is to search and develop a COX₂ inhibitor with better therapeutic effects and lower side-effects.

The inventors, after extensive studies, have surprisingly found that 4-nitro-2-(4'-methoxy-phenoxy)-methanesulfonyl aniline and its derivatives exhibit superior anti-inflammatory and analgesic effects, as compared with nimesulide, a known representative compound of this kind.

Therefore, in a first aspect, the present invention relates to 4-nitro-2-(4'-methoxy-phenoxy)-methanesulfonyl aniline of formula (1) 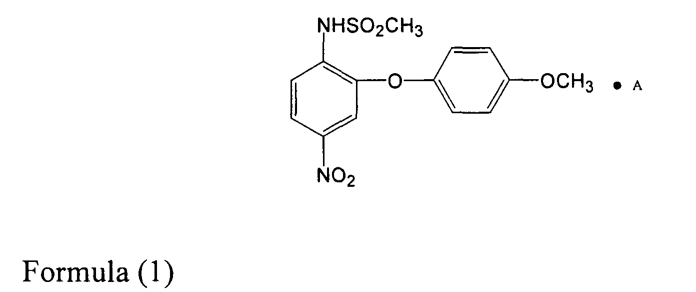 or pharmaceutically acceptable salts, solvates or hydrates thereof, where
A is present or absent, and represents a pharmaceutically acceptable inorganic or organic base, or a basic amino acid.

In another aspect, the present invention relates to a mixture of 4-nitro-2-(4'-methoxy-phenoxy)-methanesulfonyl aniline, with povidone, phospholipid or cyclodextrin.

The present invention further relates to a pharmaceutical composition for the prevention or treatment of various inflammation, pain, and the like, comprising 4-nitro-2-[(4'-methoxy)-phenoxy]-methanesulfonyl aniline, or a pharmaceutically acceptable salt, solvate, hydrate thereof, or a mixture thereof with povidone, phospholipid, or cyclodextrin, and a pharmaceutically acceptable carrier.

The present invention, in a further aspect, relates to the use of 4-nitro-2-(4'-methoxy-phenoxy)-methanesulfonyl aniline, or a pharmaceutically acceptable salt, solvate, hydrate thereof, or a mixture thereof with povidone, phospholipid, or cyclodextrin, in the preparation of non-steroid anti-inflammatory analgesic drugs.

The present invention also relates to a method for fighting against inflammation and pain, comprising administering to a patient in need thereof 4-nitro-2-(4'-methoxy-phenoxy)-methanesulfonyl aniline, or a pharmaceutically acceptable salt thereof, or a mixture thereof with povidone, phospholipid, or cyclodextrin.

According to the present invention, a pharmaceutically acceptable salt of 4-nitro-2-(4'-methoxy-phenoxy)-methanesulfonyl aniline, as used herein, refers to a salt formed by 4-nitro-2-(4'-methoxy-phenoxy)-methanesulfonyl aniline with a pharmaceutically acceptable inorganic or organic base, or a basic amino acid, for example, a salt with trans-4-methyl-cyclohexylamine, trans-4-tert-butyl-cyclohexylamine, arginine, or lysine.

According to the present invention, the mixture of 4-nitro-2-(4'-methoxy-phenoxy)-methanesulfonyl aniline with povidone, phospholipid, or cyclodextrin in the present invention can be in various forms. For example, a mixture of 4-nitro-2-(4'-methoxy-phenoxy)-methanesulfonyl aniline and povidone can be in the form of an associate of 4-nitro-2-(4'-methoxy-phenoxy)-methanesulfonyl aniline with povidone; a mixture of 4-nitro-2-(4'-methoxy-phenoxy)-methanesulfonyl aniline and phospolipid can be in the form of a complex of 4-nitro-2-(4'-methoxy-phenoxy)-methanesulfonyl aniline with phospholipid; and a mixture of 4-nitro-2-(4'-methoxy-phenoxy)-methanesulfonyl aniline with cyclodextrin can be in the form of an inclusion of 4-nitro-2-(4'-methoxy-phenoxy)-methanesulfonyl aniline and cyclodextrin.

According to the present invention, the povidone used in the present invention can be various kinds of commercially available povidones, such as povidone K30 (PVP K30, for short); and the cyclodextrin used in the present invention can be various kinds of commercially available cyclodextrins, such as α-, β-or γ - cyclodextrin.

According to the present invention, the pharmaceutically acceptable carrier, as used herein, refers to those substances that have no adverse effects on the active ingredient of the medicament, such as excipients, additives, disintegrants, adhesives, or the like well known in the art.

According to the present invention, the inflammation and pain, as used herein, include, but not limited to: osteoarthritis, respiratory tract inflammation, tumor, thromboangiitis, postoperative pain, dysmenorrhea, inflammation of the ear, nose, and throat, post-traumatic pain, fever, and the like.

According to the present invention, 4-nitro-2-(4'-methoxy-phenoxy)-methanesulfonyl aniline can be administered alone or in the form of a pharmaceutical composition. The pharmaceutical composition of the present invention can be administered orally, parenterally, or topically, and the dosage form can be tablet, capsule, drop, injection, suppository, patch, ointment, and other formulations for oral administration, injection and topical application.

According to the present invention, the pharmaceutical composition can be prepared by well-known methods in the art, e.g. by mixing the compound of formula (1) with other drugs or with a pharmaceutically acceptable carrier.

According to the present invention, the mixture or pharmaceutically acceptable salt of 4-nitro-2-(4'-methoxy-phenoxy)-methanesulfonyl aniline can be prepared by mixing 4-nitro-2-(4'-methoxy-phenoxy)-methanesulfonyl aniline (S₆) with povidone-serial compounds, phospholipid, cyclodextrin, trans-4-methyl- cyclohexylamine, trans-tert-butyl-cyclohexylamine, or a basic amino acid.

### Description of figures

Fig 1 shows the X-ray diffraction pattern of S₆.
Fig 2 shows the X-ray diffraction pattern of S₆ PM1.

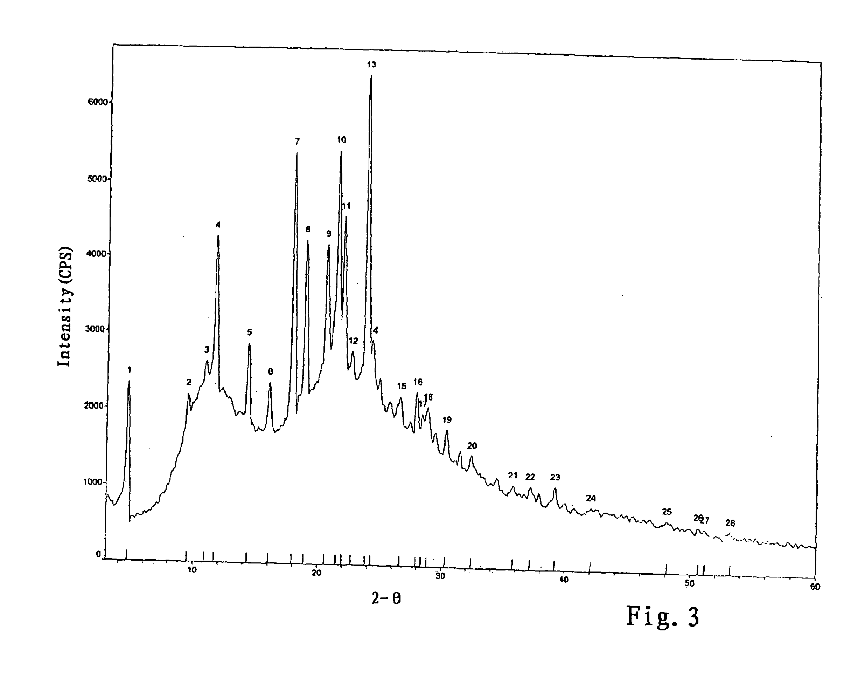

Fig 3 shows the X-ray diffraction pattern of S₆ PM3.

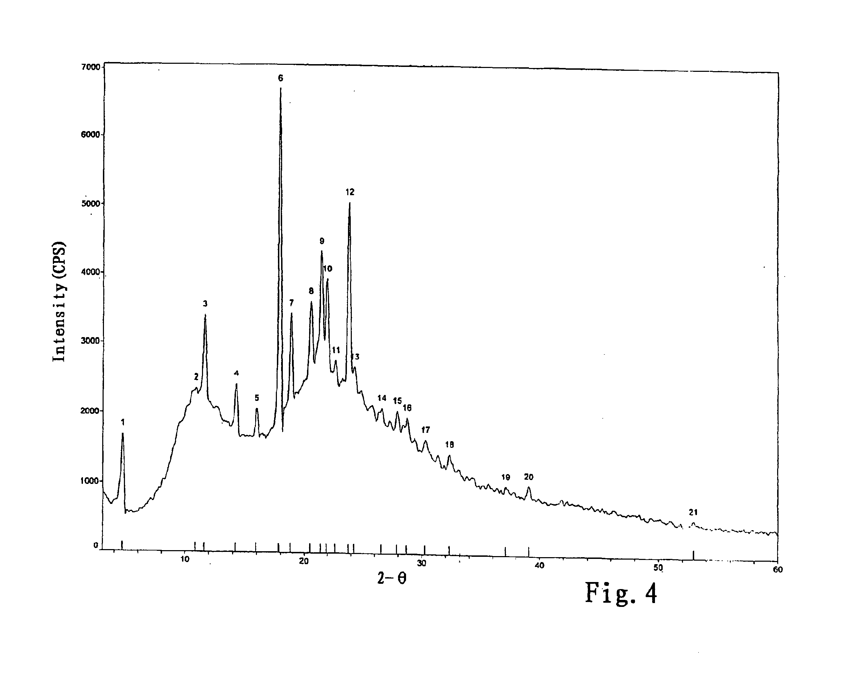

Fig 4 shows the X-ray diffraction pattern of S₆ PM5.

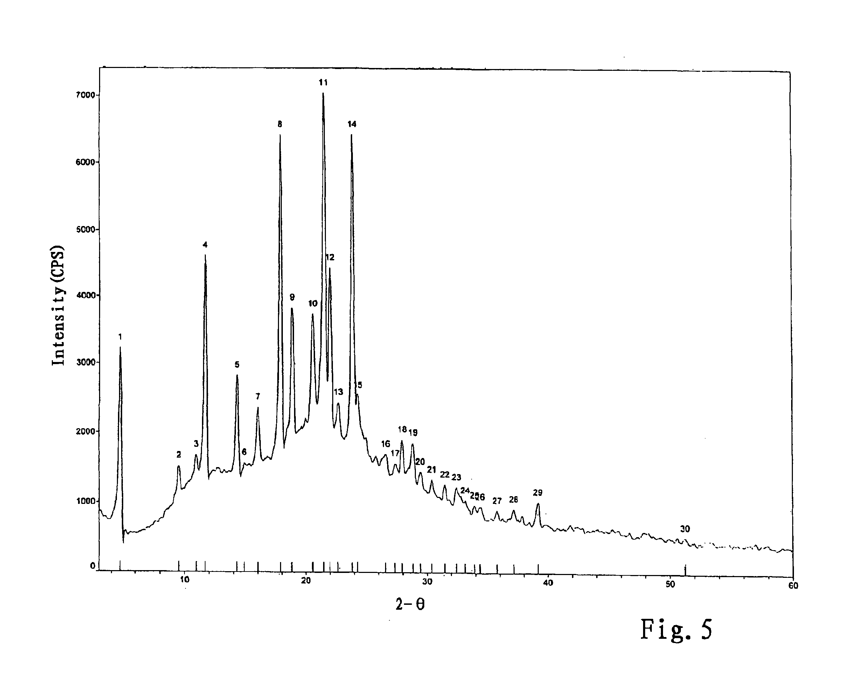

Fig 5 shows the X-ray diffraction pattern of S₆ K30-1.

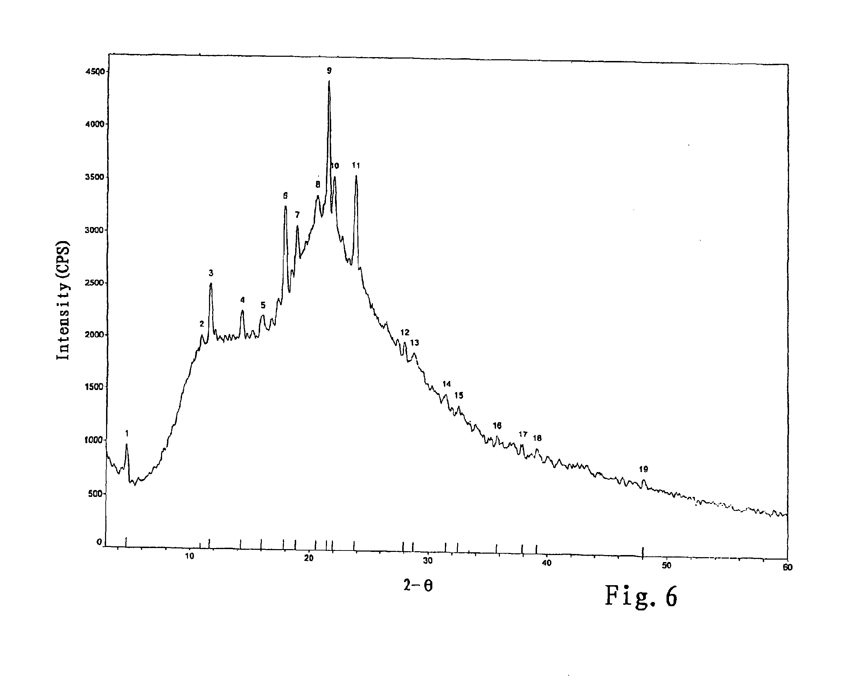

Fig 6 shows the X-ray diffraction pattern of S₆ K30-3.

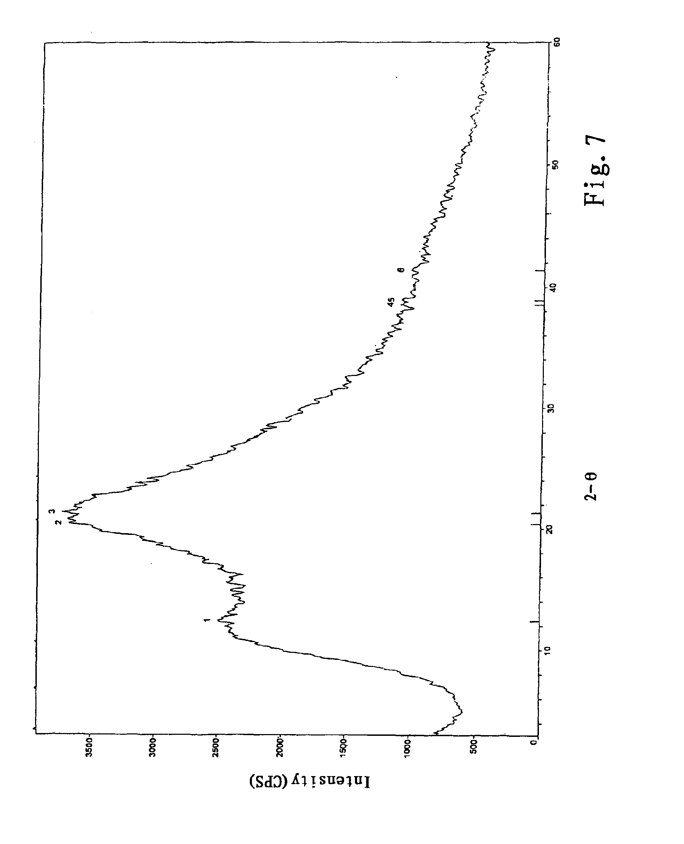

Fig 7. shows the X-ray diffraction pattern of S₆ K30-5.

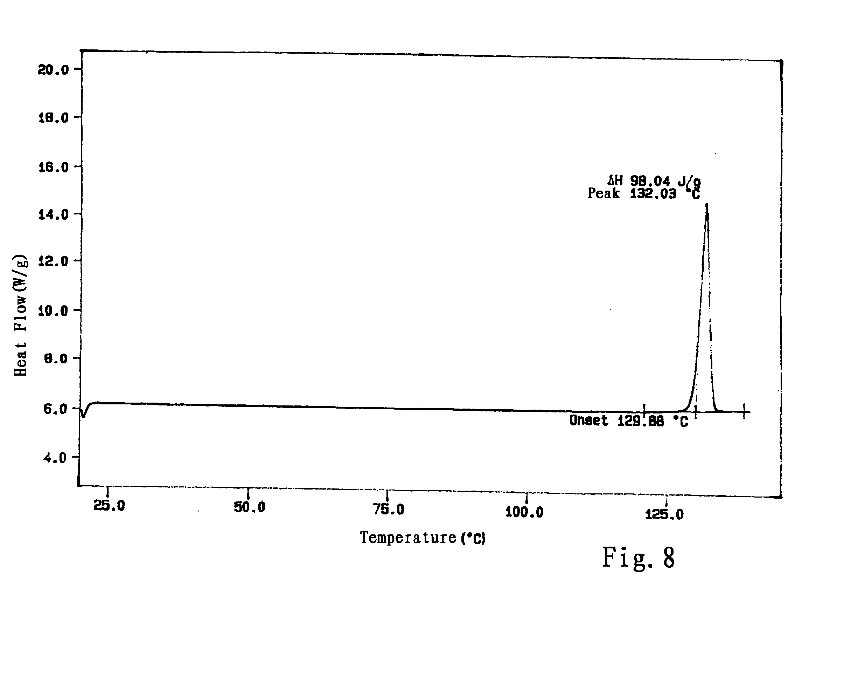

Fig 8 is the DSC graph of S₆.

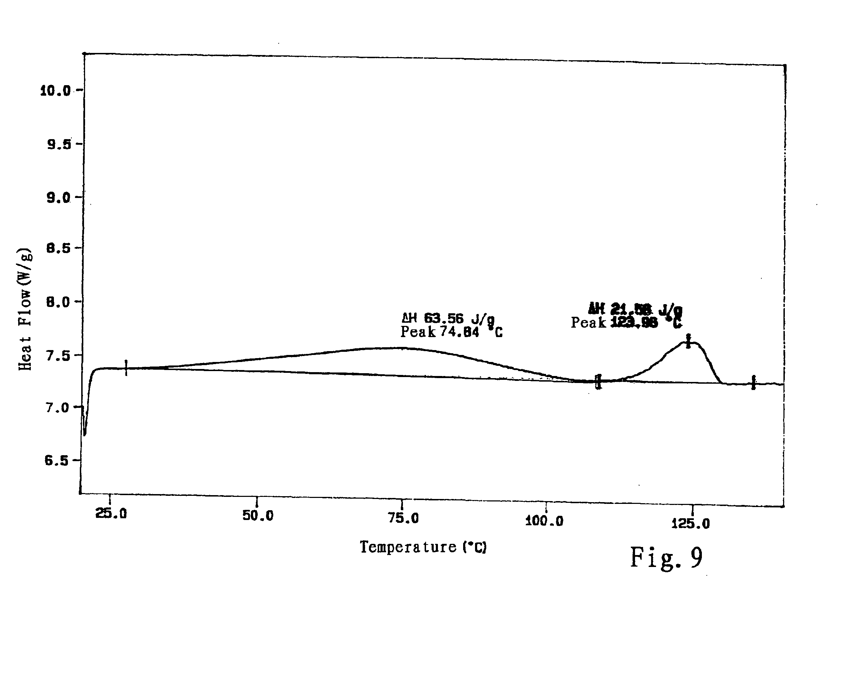

Fig 9 is the DSC graph of S₆ K30-1.

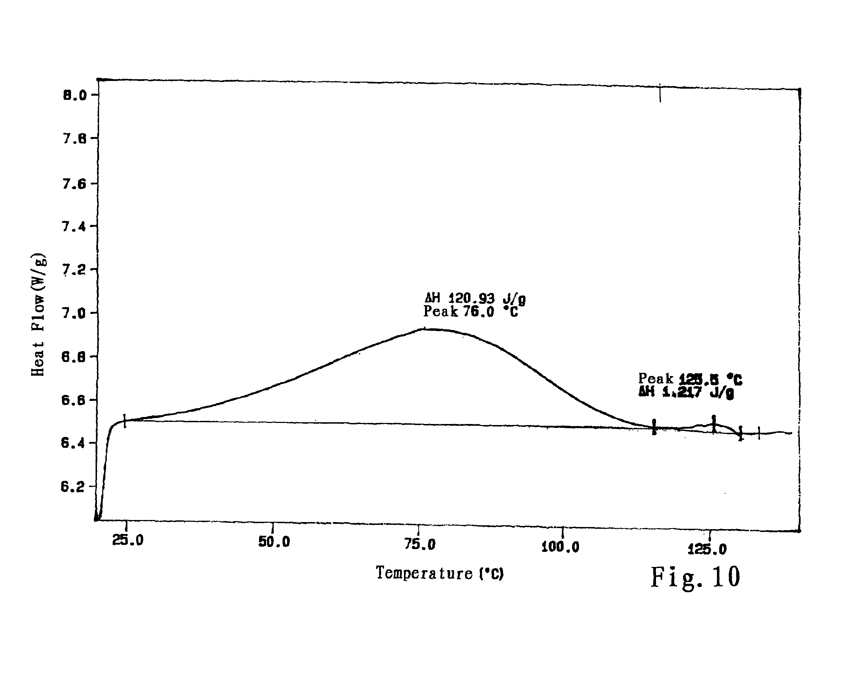

Fig 10 is the DSC graph of S₆ K30-3.

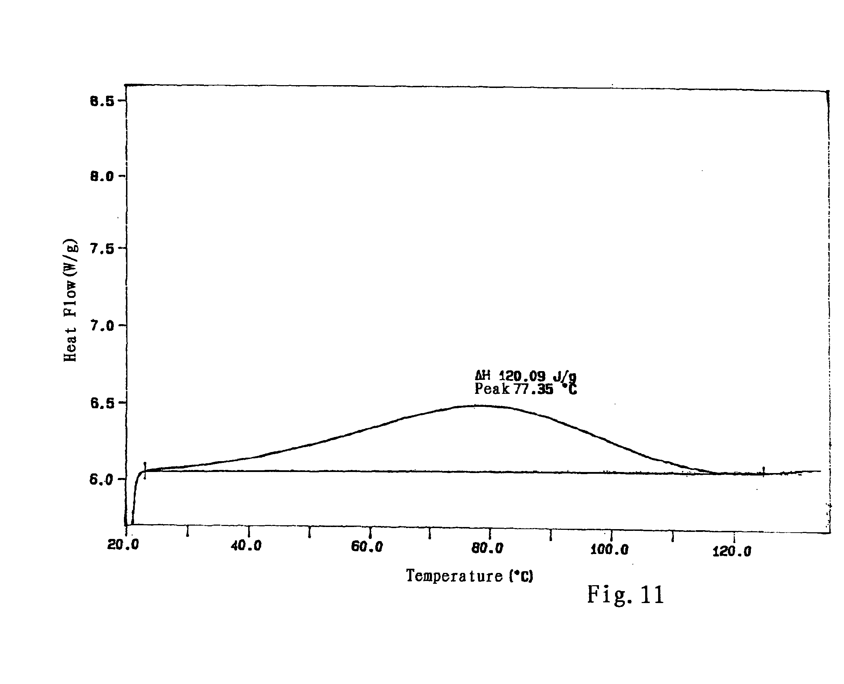

Fig 11 is the DSC graph of S₆ K30-5.

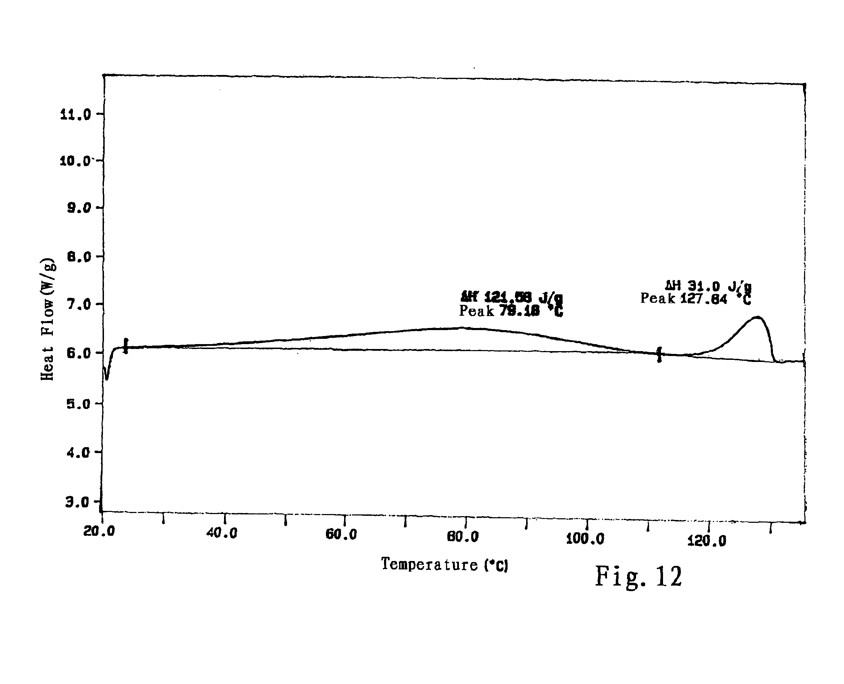

Fig 12 is the DSC graph of S₆ PM1.

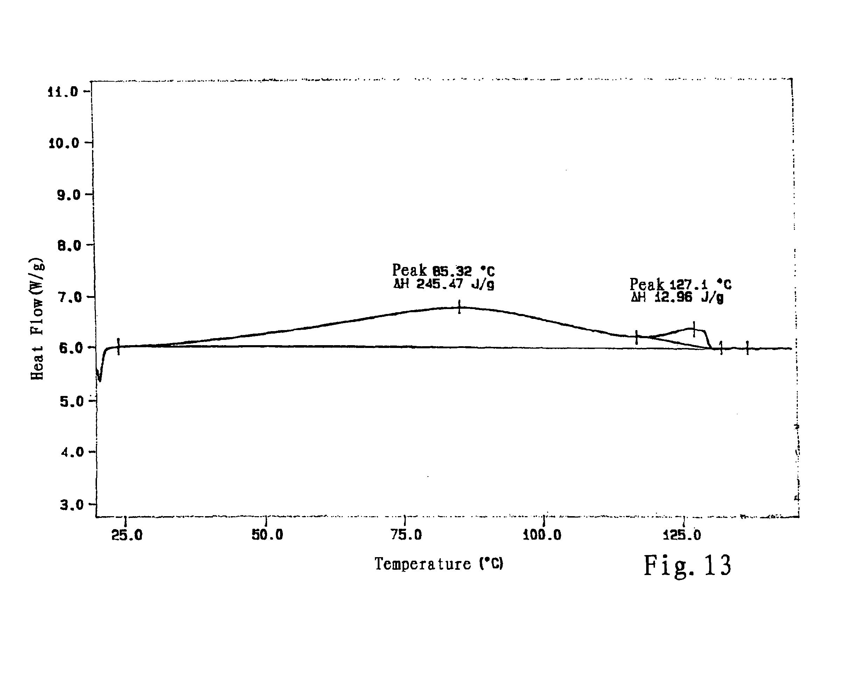

Fig 13 is the DSC graph of S₆ PM3.

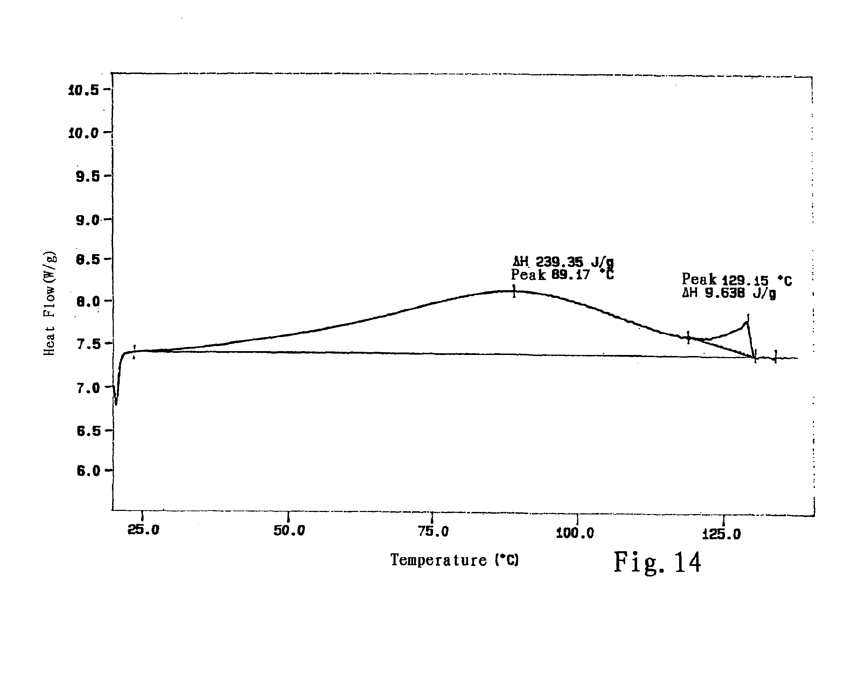

Fig 14 is the DSC graph of S₆ PM5.

### Mode of carrying out the invention

The following examples will illustrate the present invention in detail, but in no ways limiting the claims of the present invention.

### Preparation Example 1. Preparation of 2-(4'-methoxy-phenoxy)-methanesulfonyl aniline

### 1. Preparation of 2-(4'-methoxy-phenoxy)-nitrobenzene(A)

16g (0.129 mol) of para-methoxyphenol and 8g (0.143 mol) of potassium hydroxide were weighed and placed into a 50ml three-necked flask equipped with magnetic stirrer, thermometer and condensation tube. The reaction was heated to dissolve the solid. After brief cooling, about 1g of active copper and 15.8g (0.1mol) of 1-chloro-2-nitrobenzene were added, and heating was continued at reflux for 1 hour, keeping the internal temperature around 150°C. After the completion of the reaction, at 80°C, the reaction was poured into 300ml of 3% NaOH and left overnight. Yellow crystals separated out and were subjected to suction filtration, washed with distilled water to neutrality, and dried at room temperature, to give 17.4g of crude product.

17.4g of the above crude product was dissolved under heating in 200ml absolute ethanol. 6g of activated carbon was added and the reaction was refluxed for 5-10 min for decoloration. The reaction was filtered while hot, and the filtrate was cooled thoroughly for the product to separate out. After suction filtration and drying, 16.6g of (A) as a pale yellow solid was obtained. m.p.: 75-77°C. Thin layer chromatography: (developer: ethyl acetate/ petroleum ether 1:5).

### 2. Preparation of 2-(4'-methoxy-phenoxy) -aniline(B)

(A) was dissolved in q.s. ethyl acetate, and subjected to catalytic hydrogenation in the presence of Raney Ni, at 40°C, under 10kg pressure. After the completion of the reaction, the catalyst in the hydrogenation reaction was filtered off. The filtrate was concentrated under reduced pressure, to obtain a sticky product. The latter was dissolved in 10% HCl solution, adjusted to pH 4-5, filtered, and the filtrate was alkalified to pH 9-10 with 25% NaOH solution under cooling and stirring. The reaction was allowed to stand, and the organic phase separated. The aqueous phase was extracted twice with ethyl acetate. The combined organic phases were dried over anhydrous magnesium sulfate, filtered, and the filtrate concentrated, to give 14g of (B) as a sticky product.

### 3. Preparation of 2-(4'-methoxy-phenoxy)-methanesulfonyl aniline.

10g of (B) and 10ml anhydrous pyridine were added into a 100ml four-necked flask equipped with stirrer, thermometer, condensation tube and dropping funnel, heated to an internal temperature of 75°C. 6g of methanesulfonyl chloride was added slowly, keeping the internal temperature at 85-90°C, and the reaction was continued for one hour. After the completion, the reaction was poured into 18% aquesous HCl, filtered, and the filtrate was extracted three times with ethyl acetate, washed with water to neutrality. After drying over anhydrous magnesium sulfate overnight, the reaction was filtered, and ethyl acetate was evaporated off under reduced pressure. The reaction was left under cooling to give the crude 2-(4'-methoxy-phenoxy)-methanesulfonyl aniline.

The above crude product was dissolved under heating in 135ml of 95% ethanol. 4g of activated carbon was added and the mixture was refluxed for 5-10 min for decoloration, followed by filtration while hot, and the filtrate was cooled thoroughly for the product to separate out. Suction filtration followed by drying gave 9g of 2-(4'-methoxy-phenoxy)-methanesulfonyl aniline, as white crystals. m.p. 81-83 °C. Thin-layer chromatography: (developer: ethyl acetate/petroleum ether 1:5). E.A.: C₁₄H₁₅NO₄S, MW: 293.39

| | C (%) | H (%) | N (%) |
|---|---|---|---|
| Calculated | 57.32 | 5.15 | 4.77 |
| Found | 57.13 | 5.04 | 4.70 |

### Example 1. Preparation of 4-nitro-2-[(4'-methoxy)-phenoxy]-methanesulfonyl aniline and its derivatives.

### I.Preparation of 4-nitro-2-[(4'-methoxy)-phenoxy]-methanesulfonyl aniline (S₆) and its mixtures or associates with PVP_{K30} (S₆ K30-1, S₆ K30-3, S₆ K30-5)

3.6g (0.01227 mole) of 2-(4'-methoxy-phenoxy)-methanesulfonyl aniline, 30ml of glacial acetic acid and 12ml of acetic anhydride were placed in a 100ml three-necked flask equipped with stirrer, thermometer, and dropping funnel, stirred, and heated to a temperature between. 85 and 90 °C. 3g of nitric acid was slowly added dropwise. After standing under cooling, yellow crystals separated out, and were subjected to column chromatography on silica gel, eluting with dichloromethane. The fraction with R_{f}= 0.796 was collected. The combined eluates were concentrated under reduced pressure, to give a pale yellow solid, which was recrystallized from ethanol to obtain 3.5g (84.3% yield) of (S₆) as pale yellow crystals. M.p. 130-132°C. Molecular formula: C₁₄H₁₄N₂O₆S. Molecular weight: 338.33.

| Elemental Analysis: | | | | | | |
|---|---|---|---|---|---|---|
| calculated | C | 49.70% | H | 4.70% | N | 8.28% |
| found | C | 49.62% | H | 4.11% | N | 8.07% |

A solution of 100mg S₆ in absolute ethanol was added under stirring to a solution of 100mg, 300mg, or 500mg PVP_{K30} in ethanol, respectively, and a yellow clear solution was obtained. Then the solution was concentrated under reduced pressure at 60 °C to obtain a yellow oily product, which was cooled with ice and triturated with diethyl ether, to give a yellow solid. After filtering and drying under vacuum, a yellow powder was obtained, designated as S₆K30-1, S₆K30-3 and S₆K30-5, respectively.

100mg solid powder of S₆ was mixed with 100mg, 300mg or 500mg solid powder of PVP_{K30}, and triturated to obtain a homogenous mixture, designated as S₆PM1, S₆PM3 and S₆PM5, respectively.

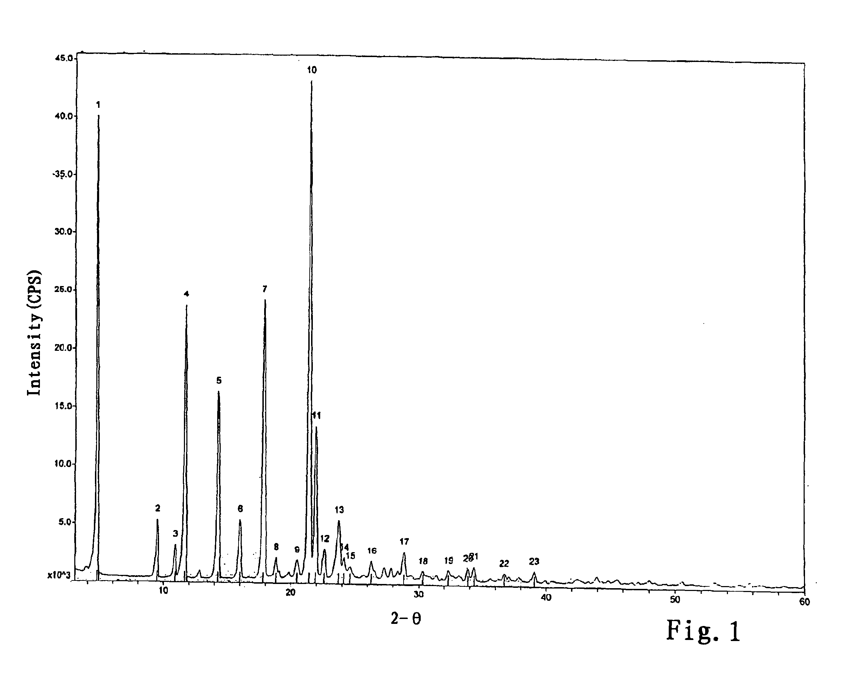

### Results of differential scanning calorimetric analysis (See Fig 1. DSC curve)

From the DSC curve it can be seen that there is a sharp endothermic peak at 132.03 °C for S₆ (melting point). The endothermic peak of PVP_{K30} is at 86.55 °C. For the physical mixtures of S₆ with PVP_{K30}, S₆PM1, S₆PM3 and S₆PM5, there are endothermic peaks of S₆ at 127.84°C, 127.1 °C and 129.15°C, respectively, whereas in the DSC curves of S₆K₃₀₋₁ and S₆K₃₀₋₃, the endothermic peak of S₆ was also present, but with a shift to the left to different extents. In the DSC curve of S₆K₃₀₋₅, the endothermic peak of S₆ completely disappeared, indicating that the S₆ crystal is completely inhibited by PVPK₃₀, resulting in forming an amorphous substance.

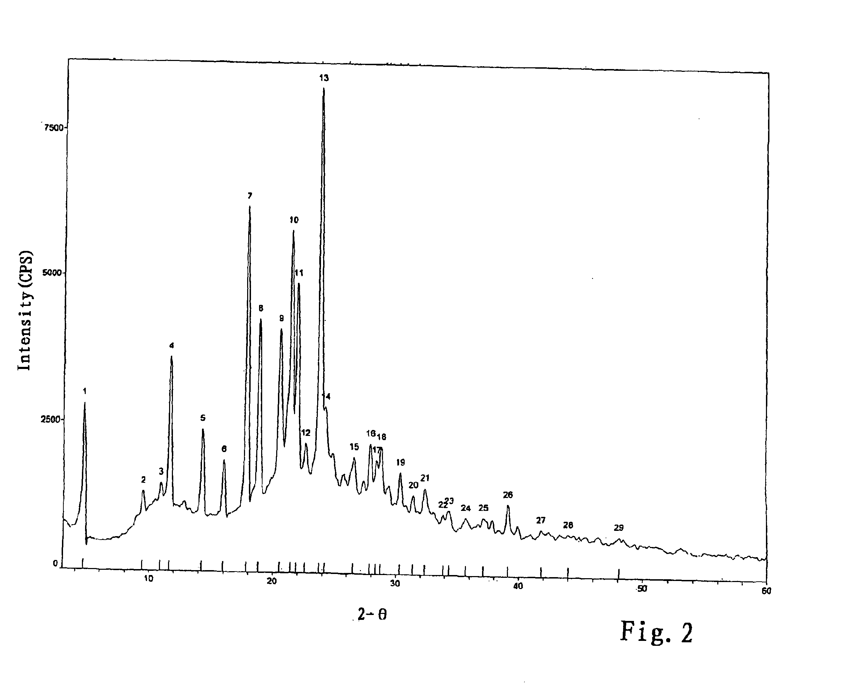

The results of X-powder diffraction (Fig 2) showed that for S6, there were a series of characteristic diffraction peaks at 4.7° -39.1° , whereas for PVP_{K30}, there is no characteristic peak between 4.7° -39.1° . For S₆PM1, S₆PM3, S₆PM5, S₆K30-1 and S₆K30-3, diffraction peaks of S₆ crystal were still present, but the peak heights were greatly reduced. Only in the X-powder diffraction pattern of S₆K30-5, the diffraction peaks of S₆ crystal were completely absent, which was consistent with the results of DSC analysis.

The above results indicate that S₆ can associate with PVP_{K30} via hydrogen bond to form a new amorphous substance.

### II. Preparation of 4-nitro-2-[(4'-methoxy)-phenoxy]-methanesulfonyl aniline, trans-4-methyl-cyclohexylamine salt (S₉).

6.8g of 4-nitro-2-(4'-methoxy-phenoxy)-methanesulfonyl aniline was dissolved in 40ml of dichloromethane, and 3.2g of trans-4-methylcyclohexylamine was added dropwise under stirring at room temperature. The reaction was heated at reflux for 1 hour, and yellow needle crystals separated out. The reaction was left to cool, and the crystals were collected by filtration, and washed with ethanol for 3 times. After drying under vacuum at 40°C, 6.7g of 4-nitro-2-(4'-methoxy-phenoxy)- methanesulfonyl aniline trans-4-methylcyclohexylamine salt was obtained. M.p.: 159-161 °C.
Molecular formula: C₂₁H₂₉N₃O₆S.
Molecular weight: 451.53

| Elemental Analysis: | | | | | | |
|---|---|---|---|---|---|---|
| calculated | C | 55.86% | H | 6.47% | N | 9.31% |
| found | C | 55.88% | H | 6.40% | N | 9.12% |

### Example 2: Biological evaluation of 4-nitro-2-[(4'-methoxy)-phenoxy] -methanesulfonyl aniline derivatives

### Anti-inflammatory and analgesic effects of 4-nitro-2-[(4'-methoxy)-phenoxy] -methanesulfonyl aniline derivatives (S₆ and S₉) in mice

In the following biological experiments, the analgesic effects of the compounds on acetate-induced body twisting in mice and their effects against carrageenin-induced paw inflammation in mice were observed, and their therapeutic effects were evaluated, using nimesulide as the positive control. The results are listed in Table 1-5.

**Table 1**

| Effects of orally administered S₆ and S₉ against paw inflammation induced by carrageenin in mice | | | |
|---|---|---|---|
| Drug | Dose (mM/Kg) | Number of animals | Paw Swelling mm (M±SD) |
| | | | at 3 hours |
| Control (DMSO) | 0.05ml/20g | 16 | 3.56±0.81 |
| Nimesulide | 0.243 | 8 | 2.10± 1.39** |
| S₆ | 0.243 | 8 | 0.58±0.67** |
| S₉ | 0.243 | 8 | 1.18±0.65** |

| | | | |
|---|---|---|---|
| ** P<0.01 vs. DMSO control | | | |

**Table 2**

| Effects of orally administered S₆ and S₆K30-5 against paw inflammation induced by carrageenin in mice | | | |
|---|---|---|---|
| Drug | Dose (mM/Kg) | Number of animals | Paw Swelling mm (M±SD) |
| | | | at 6 hours |
| Control (DMSO) | 0.05ml/20g | 12 | 4.20±0.65 |
| Nimesulide | 0.485 | 12 | 2.58±1.20** |
| S₆ | 0.485 | 12 | 1.91 ±0.66** |
| S₆K30-5 | 0.485 | 12 | 1.50±0.95** |

| | | | |
|---|---|---|---|
| **P<0.01 vs. DMSO control | | | |

**Table 3**

| Effects of orally administered S₆ against acetate-induced body twisting in mice | | | |
|---|---|---|---|
| Drug | Dose (mM/Kg) | Number of animals | No. of body-twistings (M±SD) |
| | | | at 1 hour |
| Control (DMSO | 0.05ml/20g | 10 | 48.94±11.9 |
| Nimesulide | 0.97 | 10 | 14.8±11.7** |
| S₆ | 0.485 | 5 | 11.4±5.3** |
| | 0.97 | 10 | 10.8±11.4** |

| | | | |
|---|---|---|---|
| **P<0.01 vs. DMSO control | | | |

**Table 4**

| Effects of orally administered S₉ against acetate-induced body twisting in mice | | | |
|---|---|---|---|
| Drug | Dose (mM/Kg) | Number of animals | No. of body-twisting (M±SD) |
| | | | at 3 hours |
| Control (DMSO) | 0.05ml/20g | 12 | 43.4±12.4 |
| Nimesulide | 0.485 | 8 | 11.1±9.7** |
| S₉ | 0.485 | 8 | 6.7±6.2** |

| | | | |
|---|---|---|---|
| *P<0.05, **P<0.01 vs. DMSO control | | | |

**Table 5**

| Effects of orally administrated S6K30-5 against acetate-induced body twisting in mice | | | |
|---|---|---|---|
| Drug | Dose (mg/Kg) | Number of animals | No. of body-twisting (M±SD) |
| | | | at 6 hours |
| Control (DMSO) | 0.05ml/20g | 12 | 29.84±11.99 |
| Nimesulide | 0.97 | 12 | 21.00±19.09 |
| S6K30-5 | 0.97 | 12 | 4.81±5.34** |

| | | | |
|---|---|---|---|
| **P<0.01; *P<0.05 vs. DMSO control | | | |

The experimental results of paw inflammation induced by carrageenin in mice show that the anti-inflammatory effects of S₆, S₉ and S₆K30-5 are all significantly stronger than that of nimesulide, wherein, the anti-inflammatory effects of S₉ (0.243 mM/kg) at 3 hours are 2 times stronger than nimesulide (0.243mM/kg), S₆ (0.243mM/kg) about 4 times stronger than nimesulide (0.243mM/kg). The anti-inflammatory effects of S₆K30-5 (0.485mM/kg) at 6 hours are about 2 times as strong as nimesulide.

The experimental results of analgesic effects on body-twisting induced by acetic acid in mice show that the analgesic effects of S₆, S₉ and S₆K30-5 are all significantly stronger than nimesulide, wherein, the analgesic effects of S₆ and S₉ (0.485mM/kg) are about 2 times as strong as nimesulide (0.97mM/kg), and the analgesic effects of S₆ K30-5 at 6 hours are about 4-5 times stronger than nimesulide, with a longer duration as well.

The results of acute oral toxicity of 4-nitro-2-[(4'-methoxy)-phenoxy]-methanesulfonyl aniline derivatives (S₆, S₆K30-5 and S₉) in mice (Table 6)

**Table 6**

| Comparison of acute toxicity of S₆, S₆K30-5 and S₉ orally administered in mice | |
|---|---|
| Drug | LD₅₀ value (95% C.L.) |
| | mg/kg |
| Nimesulide | 643.9 |
| S₆ | 2355.5 |
| S₆K30-5 | 6711.6** |
| S₉ | 1223.3 |

| | |
|---|---|
| ** corresponds to 1118.6 mg/kg S₆ | |

The experimental results of acute toxicity of S₆, S₆K30-5 and S₉ orally administered in mice show that S₆ has the lowest toxicity (LD₅₀=2355.5mg/kg), which is about 4 times lower than that of nimesulide. The toxicity of S₉ and S₆K30-5 is about 2 times lower than that of nimesulide. Moreover, no evident pathological changes were found in the internal organs of survived animals upon anatomic examination.

The experimental results of anti-inflammatory and analgesic effects, and of toxicity show that 4-nitro-2-[(4'-methoxy)-phenoxy]-methanesulfonyl aniline and its derivatives exhibit anti-inflammatory and analgesic effects that are significantly stronger than those of nimesulide, with a long duration of action and low toxicity.

According to the present invention, the above compounds can be formulated into enteral or parenteral preparations by known methods, such as tablets, capsules, granules, injections, suppository, drops, liniments, for oral, topical administration, injection, or the like.

The above experimental results indicate that 4-nitro-2-[(4'methoxy)-phenoxy]-methanesulfonyl aniline (S₆) and its derivatives (such as S₆K30-5 and S₉) possess anti-inflammatory and analgesic effects that are significantly stronger than nimesulide, while their toxicity is significantly lower, and the duration of action is longer.

## Claims

1. A compound of formula (I) 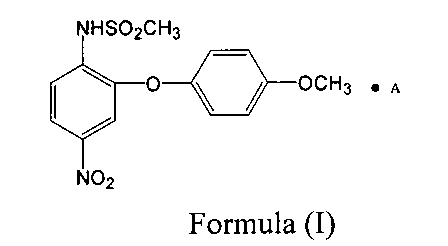 or salts, solvates or hydrates thereof,
wherein A is present or absent and represents a pharmaceutically acceptable inorganic or organic base, or a basic amino acid.

2. The compound according to claim 1, wherein A is trans-4-methyl-cyclohexylamine or trans-4-tert-butyl-cyclohexylamine.

3. The compound according to claim 1, wherein A is arginine or lysine.

4. A mixture of the compound of formula (I) wherein A is absent with povidone, phospholipid, or cyclodextrin.

5. The mixture according to claim 4, wherein said mixture is an associate of said compound of formula (I) with povidone.

6. The mixture according to claim 4, wherein said mixture is a complex of said compound of formula (I) with phosplolipid.

7. The mixture according to claim 4, wherein said mixture is an inclusion of said compound of formula (I) and cyclodextrin.

8. A pharmaceutical composition, comprising a compound according to any one of claims 1 to 3 or a mixture according to any one of claims 4-7, and a pharmaceutically acceptable carrier.

9. The pharmaceutical composition according to claim 8, wherein said composition is in the form of oral preparation, injection, suppository, drop, or preparation for external use.

10. Use of 4-nitro-2-[(4'-methoxy)-phenoxy]-methanesulfonyl aniline or pharmaceutically acceptable salts thereof, or a mixture thereof with povidone, phospholipid or cyclodextrin in the preparation of anti-inflammatory analgesic drugs.
